(19) Europäisches Patentamt / European Patent Office / Office européen des brevets

(11) **EP 4 389 159 B1**

(12) **EUROPEAN PATENT SPECIFICATION**

(45) Date of publication and mention of the grant of the patent:
**11.02.2026 Bulletin 2026/07**

(21) Application number: **22215556.6**

(22) Date of filing: **21.12.2022**

(51) International Patent Classification (IPC):
**A61L 2/08** (2026.01)    **A61L 2/10** (2026.01)
**A61L 2/24** (2006.01)    **A61L 9/20** (2006.01)

(52) Cooperative Patent Classification (CPC):
**A61L 2/10; A61L 2/084; A61L 2/24; A61L 9/205;**
A61L 2202/25; A61L 2209/11

(54) **MEDICAL DEVICE FOR DISINFECTING A VOLUME OF A HOSPITAL INDOOR ENVIRONMENT AND METHOD FOR SAID DISINFECTION**

MEDIZINISCHE VORRICHTUNG ZUR DESINFEKTION EINES VOLUMENS EINER KRANKENHAUSINNENRAUMUMGEBUNG UND VERFAHREN ZUR DESINFEKTION

DISPOSITIF MÉDICAL POUR DÉSINFECTER UN VOLUME D'UN MILIEU INTÉRIEUR HOSPITALIER ET PROCÉDÉ POUR LE DÉSINFECTER

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Validation States:
**MD**

(43) Date of publication of application:
**26.06.2024 Bulletin 2024/26**

(73) Proprietor: **TERRA VENTURES S.R.L.**
**judet Timis (RO)**

(72) Inventor: **NECULAI, Miron**
**mun. Timisoara (RO)**

(74) Representative: **Vasilescu, Raluca**
**Cabinet M. Oproiu**
**Patent and Trademark Attorneys**
**42, Popa Savu Street**
**Sector 1, P.O. Box 2-229**
**011434 Bucharest (RO)**

(56) References cited:
CN-U- 216 897 686     DE-T2- 69 637 493
US-A- 6 139 803     US-A1- 2016 249 760

**Description**

Field of the invention

The present invention refers to a medical device and a method for disinfecting hospital indoor environments contaminated with germs.

In particular, the present invention refers to a medical device comprising a portable activation panel, coated with a film of titanium dioxide $TiO_2$ nanoparticles, acting as photocatalyst, one irradiating lamp, and an electronic control unit; and to a computer-implemented method of configuration and operation of the medical device of the invention.

Terms to be used in the invention

Throughout this invention, the term "hospital indoor environments" refers to all kind of indoor spaces of medical care units that can generate nosocomial micro-organisms, such as: hospitals, nursing houses for elderly people or for people with disabilities, medical centers, diagnostic laboratories, etc. In this invention, the term "hospital indoor environments" includes the air, as well as all the objects placed within said indoor environments. Geometrically speaking, the hospital indoor environments are closed environments, spatially delimited by structural elements of the building: walls, floors and ceilings.

The term "germs", alternatively called "nosocomial micro-organisms", refers to the micro-organisms that are known by the health care specialists to originate in the hospital medical care indoor environments and that can bring serious injuries to the health of the patients, including death

The term "disinfecting hospital indoor environments" refers thus to disinfecting the air and all the objects placed within said indoor environments.

The term "photocatalytic composition", alternatively called "photocatalyst", refers to a substance or a combination of substances that are activated under photoirradiation.

The term "UV light", alternatively called "UV radiation" refers to the light, alternatively called radiation within the ultra-violet light spectrum, including the three sub-domains of UV-A, UV-B and UV-C.

The term "natural light", alternatively called "daylight", refers to the light of the visible spectrum together with the UV-A spectrum.

Background of the invention

Nosocomial infections are infections generated in the hospital indoor environments caused by the nosocomial micro-organisms, including bacteria and fungi. Non-limited examples of nosocomial micro-organisms are: *Escherichia coli, Staphylococcus aureus* which is a *Methicillin-resistant (MRSA)* drug, *Streptococcus pyogenes, Candida albicans, Pseudomonas aeruginosa, Clostridium difficile, Klebsiella pneumoniae,* etc.

Dancer *et al* [01] provide an excellent presentation of the fundamental understanding of infectious diseases.

The differentiating element of the nosocomial infections as compared with other infections possibly generated by the same micro-organisms is that in case of the nosocomial infections, the ego patient gets infected during his stay in the hospital indoor environment.

Typically, the source of the infections may be the ego patient himself (his own microbiota), one or more of the other patients, one or more of the medical specialists or the source cannot be determined. For the purpose of the invention, the source of the nosocomial infection is not relevant.

They do represent a serious threat for patient's health and, in extreme cases, life, because the nosocomial micro-organisms are opportunistic micro-organisms taking advantage of the compromised health conditions of most of the patients. Some extreme examples are the nosocomial infections causing severe pneumonia, infections of the bloodstream or of the urinary tract.

The nosocomial infections spread from one patient to another by various ways, for example by air, dispersed by air currents, by direct contact between the persons, by touching infected objects (such as beds, tables, chairs, food, etc.) or by vectors such as mosquitos.

Given the various ways of transmission and the fact that many times it is difficult to assess which way each micro-organism is spreading, it is important to proceed to disinfect at a same time both the air and all the objects placed within said indoor environments.

Currently there are some disinfection methods, the most frequently used categories being listed below:

-    Disinfection of the surfaces of the objects by using disinfectants and light from the visible spectrum. In this category falls the disinfectants based on titanium dioxide $TiO_2$, as disclosed for example in Matsunaga *et al* [02],
-    Disinfection of the surfaces and/or air using devices based on UV radiation, of different wavelength, as disclosed for example in M. Takeuchi *et al* [03],

- Disinfection of the surfaces and/or air can be done with devices using combined disinfectants that have photocatalytic properties, such as the devices based on UV radiation based on titanium dioxide, as disclosed for example in Jacoby *et al* [04] and in Howard A. Foster *et al* document [05]. The latter explains that: "The ability of titanium dioxide (titania, TiO2) to act as a photocatalyst has been known for 90 years (Renz 1921), and its role in the "chalking" of paint (formation of powder on the surface) is well known (Jacobsen 1949)" [...] "The killing mechanism involves degradation of the cell wall and cytoplasmic membrane of [...] due to the production of reactive oxygen species such as hydroxyl radicals and hydrogen peroxide."

An excellent description of the principles on which the photocatalytic disinfection is based on by applying UV radiation to surfaces coated by titania, is found in said Howard A. Foster *et al* document [05].

"$TiO_2$ is a semiconductor. The adsorption of a photon with sufficient energy by $TiO_2$ promotes electrons from the valence band (evb-) to the conduction band (ecb-), leaving a positively charged hole in the valence band (hvb +; Eq. 1). The band gap energy (energy required to promote an electron) of anatase is approx. 3.2 eV, which effectively means that photocatalysis can be activated by photons with a wavelength of below approximately 385 nm (i.e. UVA). The electrons are then free to migrate within the conduction band. The holes may be filled by migration of an electron from an adjacent molecule, leaving that with a hole, and the process may be repeated. The electrons are then free to migrate within the conduction band and the holes may be filled by an electron from an adjacent molecule. This process can be repeated."

EP0630679 discloses a photocatalytic **process** for the treatment of contaminated air in an indoor space, said process comprising three steps. In the first step, at least one electric lamp is disposed to illuminate said space. In the second step, it is provided a thin film of photocatalyst of solid-state semiconductor material on at least a part of the interior surface of said space in a light receiving relationship from said lamp. In the third step, said lamp is energized whereby said thin film of photocatalyst is photoexcited. The lamp disclosed in EP0630679 is an electric lamp for general lighting applications, being capable of emitting at least a small amount of ultraviolet radiations within the wave length range from 300 nm to the wave length corresponding to the band gap energy of said semiconductor material.

According to EP0630679, the wattage of the general-application electric lamp and the distance between the photocatalytic thin film and the lamp are selected so that the total incident light intensity of UV radiation having an energy higher than the band gap energy of the semiconductor photocatalyst is 0.001-1 mW/cm$^2$, preferably, 0.01-0.1 mW/cm$^2$.

According to EP0630679- paragraph [0023], the commercial general-application electric lamps are safe and harmless since they are primarily designed to cut off harmful UV radiations having a wave length of less than 300 nm.

According to EP0630679, the wavelength of the lamp of the UV radiation is in the range of 300 to 390 nm, which corresponds to the known wavelength ranges for which titanium dioxide $TiO_2$ acts as photocatalyst when excited by said UV radiation.

US 2016/249760 discloses curtains which have fastener/s and, in some cases, strut/s arranged along a screen and systems which include a disinfection apparatus and the curtains.

The curtains include a screen configured to attenuate a majority amount of the visible light spectrum and/or a majority amount of the UVC light spectrum and one or more fasteners disposed along the screen at least 20 inches from an upper edge of the screen, wherein the one or more fasteners are configured for attaching the curtain to an object.

The disinfection apparatus includes a germicidal light source arranged within the apparatus to emit light into an ambient of a room in which the apparatus is arranged, a support structure supporting a base of the germicidal light source, and a shield attached to the Support structure. The shield extends to a first elevation at least two feet above the germicidal light source and borders at least one third of a continuous region Surrounding the germicidal light source. In addition, the shield is configured to block a majority amount of the visible light spectrum and/or a majority amount of the ultraviolet electromagnetic radiation Subtype C light spectrum.

Disadvantages of prior art

- The disinfection methods based exclusively on using disinfectants based on titanium dioxide $TiO_2$ activated by light from the visible spectrum have the main disadvantage that natural light is not sufficient to activate the photocatalyst and, more important, in the absence of natural light there is no disinfection, thus this method cannot be used as a round-the-clock disinfection,
- In order to be effective for disinfection, the majority of the UV lamps used alone for disinfection have wavelengths in the 180-280 nm range, that is exactly the wavelength range for creating ozone, the important disadvantage of which being that can cause throat irritation and other respiratory-related issues as well as injuries to the eyes of the persons or animals in the room. During the disinfection there must be no persons, animals or plants left in the room. After disinfection, ventilation is mandatory, and natural air ventilation highly recommended. The disinfection process is, thus, cumbersome.

- The disinfection methods based on irradiating with UV lamps the surfaces coated with titanium dioxide $TiO_2$, have the general common disadvantage that they cannot be adjusted to the particulars of each hospital indoor environments: operating room, wards, patient rooms, emergency room, intensive care unit, hallways, etc. The known methods from prior art provide the same level of disinfection for all types of indoor environments- be they for hospital use or not. The disinfection method of EP0630679 has, apart from the general afore-mentioned disadvantage, the following specific disadvantages: it is not adjusted to the volume of each hospital indoor environment; as the thin film of photocatalyst is applied on the interior surface of the spaces, one cannot walk away with the interior surface and move it to another place; the lamps used have a high electricity consumption and a short lifespan.

Problem to be solved by the invention

Therefore, to address the disadvantages of prior art, the technical problem to be solved by the invention is to find a versatile medical device comprising an irradiating lamp and a surface coated with a film of titanium dioxide $TiO_2$ nanoparticles suitable for disinfecting a wide variety of hospital indoor environments contaminated with germs, adaptable to the destination of each specific category of hospital indoor environment; adaptable to each specific volume of the hospital indoor environment; easy to carry it from one place to another; having low electricity consumption and a high lifespan.

Summary of the invention

In order to overcome the disadvantages of prior art, in a first aspect of the invention it is presented a medical device suitable for disinfecting a volume V of a hospital indoor environment contaminated with germs, the medical device comprising a portable activation panel PAN, having a non-porous portable activation panel PAN surface coated with a film of titanium dioxide TiO2 nanoparticles, arranged to be maintained firmly in place during said disinfecting, the portable activation panel PAN acting as photocatalyst, one irradiating lamp L, and an electronic control unit ECU,

the irradiating lamp L being placed at an adjustable activation distance d from the portable activation panel PAN, the irradiating lamp L arranged to be maintained firmly in place during said disinfecting,
the irradiating lamp L comprising at least one LED (Light Emitting Diode) having a wavelength $\lambda$ in the range of $320 \div 380$nm and a power P,
the irradiating lamp L being arranged to irradiate the portable activation panel PAN during at least one activation period over 24 hours, where each activation period is equal to or greater than a minimum number of activation hours of 3 hours,
the electronic control unit ECU being in communication with the irradiating lamp L,
the irradiating lamp L being operable by the electronic control unit ECU,

Said portable activation panel PAN is a sliding panel, a folding panel or a panel configured to be rolled up and down.

A first ratio $r_1$ is defined between an area s of the portable activation panel PAN and the volume V of said hospital indoor environment, said first ratio $r_1$ being in the range of 1:20 - 1:30.

A second ratio $r_2$ is defined as the power of said irradiating lamp transferred per unit area of the portable activation panel PAN, said second ratio $r_2$ being in the range of 16.5 - 19.5 $W/m^2$.

Based on said volume V of said hospital indoor environment, on the minimum number of activation hours, and on a selection of the first ratio r1 and of the second ratio r2, the electronic control unit ECU is arranged:

to compute the area s of the portable activation panel PAN,
to determine the adjustable activation distance **d,** the power P of the irradiating lamp L, and to send the corresponding determinations to the irradiating lamp L,
to set each activation period and the number of activation periods, and to send the corresponding settings to the irradiating lamp L,
to send power on instructions to the irradiating lamp L at the beginning of each activation period,
to send power off instructions to the irradiating lamp L at the end of each activation period.

The medical device is configured to carry out photocatalytic disinfection of said V of said hospital indoor environment for each activation period by irradiating the area s of the portable activation panel PAN by the irradiating lamp L.

In a second aspect of the invention it is presented a method of configuration and operation of the medical device of any of the claims 1 to 10, comprising the following steps carried out by the electronic control unit ECU:

S1 receiving data regarding the volume V of the hospital indoor environment contaminated with germs, the minimum number of activation hours, the selected value of the first ratio r1 and the selected value of the second ratio $r_2$,

S2 computing the area s of the portable activation panel PAN by using the first ratio,

S3 determining the adjustable activation distance $d$, the power P of the irradiating lamp L, and sending the corresponding determinations to the irradiating lamp L,

S4 setting each activation period and the number of the activation periods, and sending the corresponding settings to the irradiating lamp L,

S5 powering on the irradiating lamp L at the beginning of each activation period,

S6 powering off the irradiating lamp L at the end of each activation period.

In a third aspect of the invention it is presented a non-transitory computer-readable medium comprising instructions which, when the program is executed by the electronic control unit ECU of the invention, causes the electronic control unit ECU to carry out the steps of the method according to any preferred embodiment.

Advantages

The medical device and the method of the invention have the general advantage that it allows better disinfection of the hospital indoor environments as compared with prior art, because of its adaptability to the variety of real-life situations:

- the medical device is adaptable to the destination of each specific category of hospital indoor environment as the minimum numbers of activation hours correspond to various specific hospital indoor environments,
- the medical device is adaptable to each specific volume of the hospital indoor environment because of the definition of the first ratio,
- the portable activation panel of the device allows carrying it from one place to another, thus providing flexibility,
- the irradiating lamp L of the medical device has low electricity consumption and a high lifespan, due to the use of the LEDs,
- the time of exposure of the irradiating lamp L of the medical device is adjustable,
- the medical device allows being operated automatically according to the method of the invention, because of the presence of the electronic control unit.

List of drawings

Further special features and advantages of the present invention can be taken from the following description of an advantageous embodiment by way of the accompanying drawings:

Fig. 1 illustrates the first alternative lamp-panel preferred embodiment

Fig. 2a illustrates the second alternative lamp-panel preferred embodiment

Fig. 2b illustrates a detail of Fig. 2a

Fig. 3a illustrates the standalone individual panels

Fig. 3b illustrates the sliding panels

Fig. 3c illustrates the folding panels

Fig. 3d illustrates the rolled panels

Fig. 3e illustrates the light scattering surface.

Fig. 4a illustrates the custom irradiating lamp of the preferred example of realization

Fig. 4b illustrates the setup of the preferred example of realization

Fig 5 - Table 1 illustrates characteristics of the germs of the hospital experiment

Fig. 6 - Table 2 illustrates conclusions of the hospital experiment

Fig. 7a and Fig. 7b illustrate the absorbance spectrum of the sanitizer

Fig. 8 illustrates the Petri dishes of the university experiment

Fig. 9 shows the results of the university experiment in a graph and a table

Fig. 10 -Table 3 comparison between the results of the hospital and university experiments

Reference signs

PAN      portable activation panel, coated with a film of titanium dioxide $TiO_2$ nanoparticles
         *s* area of the portable activation panel
L         irradiating lamp
LED      light emitting diode of the irradiating lamp
*d*        adjustable activation distance
α         adjustable activation angle
θ         divergence angle
ECU      electronic control unit

Detailed description

The medical device according to the invention is suitable for disinfecting a volume V of a hospital indoor environment contaminated with germs.

The medical device according to the invention comprises a portable activation panel PAN, having a non-porous portable activation panel PAN surface coated with a film of titanium dioxide $TiO_2$ nanoparticles, acting as photocatalyst, one irradiating lamp L, and an electronic control unit ECU.

The surface of the portable activation panel PAN must be non-porous to prevent the absorption of the titanium dioxide $TiO_2$ nanoparticles into the material of the portable activation panel PAN, and thus to enhance the irradiation of said portable activation panel PAN.

The portable activation panel PAN is arranged to be maintained firmly in place during said disinfecting by detachable panel fixing means, said detachable panel fixing means being beyond the scope of the invention. If required to move the portable activation panel PAN from a first place to a second place, said portable activation panel PAN is detached from the first place by detaching the panel fixing means, moved to the second place and maintained firmly in place there by said detachable fixing means.

The features of the film of titanium dioxide $TiO_2$ nanoparticles, such as: the composition of the solution of titanium dioxide $TiO_2$, the presence or absence of doping substances in said solution, the number of film coatings and the periodicity of applying said film coatings are according to prior art.

For those hospital indoor environments provided with one or more windows allowing daylight to penetrate through said windows, in a preferred embodiment, the position of the portable activation panel PAN is facing said one or more windows, such that said daylight irradiate the portable activation panel PAN.

The irradiating lamp L is placed at an adjustable activation distance *d* from the portable activation panel PAN. Said activation distance *d* is measured in the common way, that is perpendicularly on the panel surface.

The irradiating lamp L arranged to be maintained firmly in place during said disinfecting by lamp fixing means, said lamp fixing means being beyond the scope of the invention.

The irradiating lamp L comprises at least one LED (Light Emitting Diode) having a wavelength λ in the range of $320 \div 380nm$ and a power P.

The choice of the irradiating lamp L comprising the at least one LED was made because of the intrinsic advantages of the LEDs in respect to reduced cost, compact size, lightweight, lower operating temperature, and in particular long lifetime as well as its directionality in order to obtain the maximum illumination of the portable activation panel PAN, which has the advantage that it allows use of the medical device of the invention for a larger number of hours than the lamps from prior art. The irradiating lamp L is arranged to irradiate the portable activation panel PAN during at least one activation period over 24 hours, where each activation period is equal to or greater than a minimum number of activation hours of three hours over a 24 hours period.

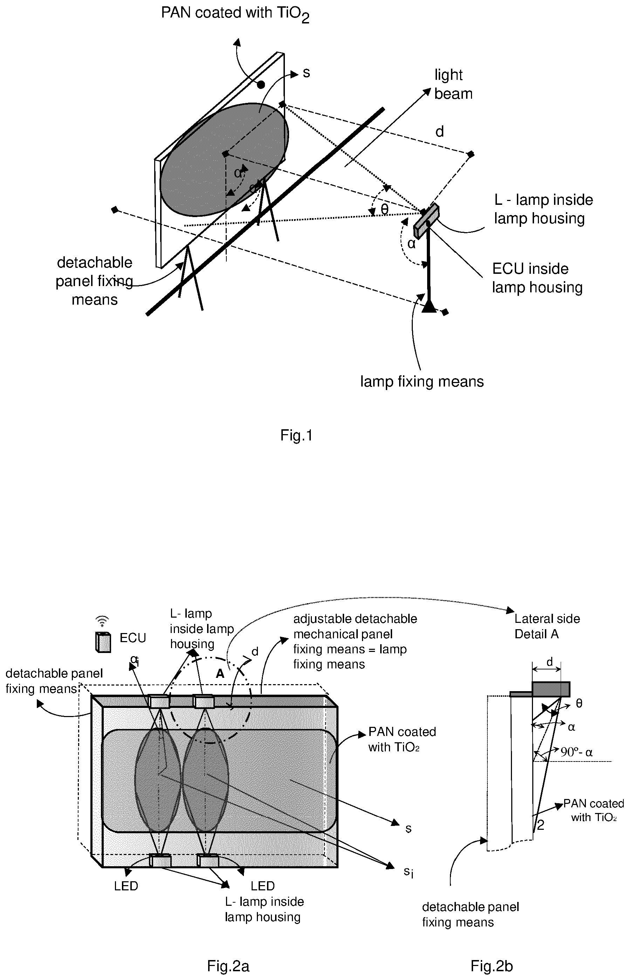

The LEDs have a divergence angle θ, as seen in Fig. 1 and Fig. 2b.

The irradiation is shown schematically in Fig. 1, Fig. 2a and Fig. 2b by representing the light beam of the irradiating lamp L. When in use, that is during the at least one activation period, the irradiating lamp L and the portable activation panel PAN are connected by the light beam.

The electronic control unit ECU is in communication with the irradiating lamp L. In one embodiment, the electronic control unit ECU and the irradiating lamp L are arranged in a single hardware entity, as represented schematically in Fig. 1, whereas in another embodiment, the electronic control unit ECU is arranged in a dedicated hardware entity communicating with one or more irradiating lamps L by wired or wireless protocols, as represented schematically in Fig. 2a. It shall be understood that the arrangement of the electronic control unit ECU represented in Fig.1 and Fig. 2a is for exemplification only and not limiting the invention.

The irradiating lamp L is operable by the electronic control unit ECU.

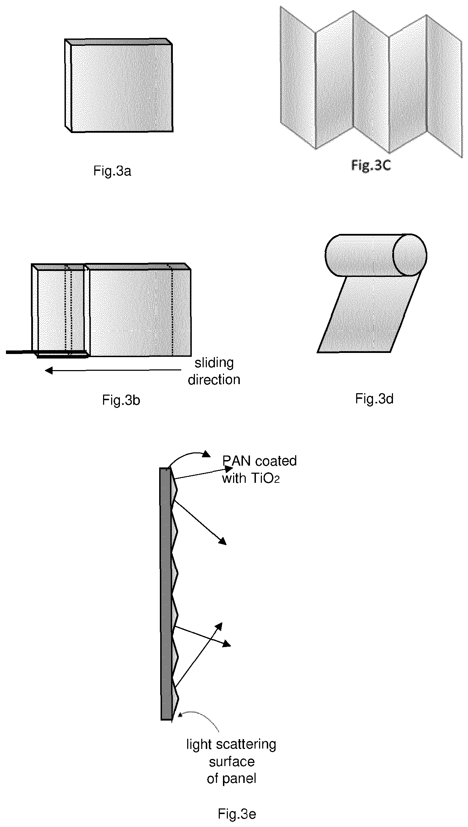

Said portable activation panel PAN is a sliding panel - as shown in Fig. 3b, a folding panel - as shown in Fig. 3d, or a panel configured to be rolled up and down - as shown in Fig.3d.

A first ratio $r_1$ is defined between an area s of the portable activation panel PAN and the volume V of said hospital indoor environment, said first ratio being in the range of 1:20 - 1:30. Mathematically speaking, the first ratio $r_1$ is written as follows:

$$r_1 = s/V \quad [\text{Eq.}1]$$

$$r_1 \in [1:20 - 1:30] \quad [\text{Eq.}2]$$

$$[s] = m^2, [V] = m^3$$

The role of the first ratio $r_1$ is to calculate which area s of the portable activation panel PAN is needed to be irradiated by the irradiating lamp L depending on the volume V of each specific hospital indoor environment to ensure the adaptability of the medical device to the specific volume of each hospital indoor environment. The irradiating lamp L is configured to irradiate the entire surface of the portable activation panel PAN. However, in order to disinfect a specific volume V, it may not be needed to irradiate the entire surface, but only a part. The area s is smaller than or equal to the total surface of the portable activation panel PAN:

$$s \leq \text{total surface of the portable activation panel PAN} \quad [\text{Eq.}3]$$

The idea to use the first ratio $r_1$ stems from the fact that the photocatalytic disinfection is volume-related, disinfecting the air and all the objects that are placed inside the volume V and not covered or placed within closed drawers/closets, etc.

The ranges of the first ratio have been determined such that to adapt the medical device of the invention to one of the categories of hospital indoor environments that is very frequent: the rooms having the room area between approximately 20 and $25m^2$- such as, but not limited to $21m^2$, $21.5m^2$, $22.m^2$, $22.5m^2$, $23m^2$, $24.9m^2$, etc. and the room height around 2.40-2.60m, the volume V being in the range between $48m^3$ and $65m^3$ included, without having the total surface of the portable activation panel PAN too large to be transported where necessary. Said total surface of the portable activation panel PAN for the volume V in the range of $48m^3$ and $65m^3$ is comprised between $1.6m^2$ and $3.25m^2$, such as but not limited to $1.7m^2$, $1.85m^2$, $1.95m^2$, $1.98m^2$, $2m^2$, $2.05m^2$, $2.5m^2$, $2.8m^2$, $3.00m^2$, $3.21m^2$, etc. For categories of hospital indoor environments having larger volumes V, more than two medical devices of the invention shall be used.

A second ratio $r_2$ is defined as the power of said irradiating lamp L transferred per unit area of the portable activation panel PAN.

Said second ratio is in the range of 16.5 - 19.5 $W/m^2$. Mathematically speaking, the second ratio $r_2$ is written as follows:

$$r_2 = P/s \quad [\text{Eq.}4]$$

$$r_2 \in [16.5 - 19.5 \ W/m^2] \quad [\text{Eq.}5]$$

$$[P] = W; [s] = m^2$$

The range of the second ratio $r_2$ was determined with the aim to optimize at the same time the efficiency of the photocatalytic disinfection, and the performance of the irradiating lamp L, having in view that, for the same power P of the irradiating lamp L, as the adjustable activation distance *d* increases, the illumination per unit area of the portable activation panel PAN by the irradiating lamp L, that is the measure of the luminous flux per unit area that reaches the portable activation panel PAN is inversely proportional to the square of said adjustable activation distance *d*.

$$E_1/E_2 = d_2{}^2/d_1{}^2 \quad [\text{Eq.}6]$$

where $d_1$ and $d_2$ are two different values of the adjustable activation distance *d*,
and where $E_1$, respectively $E_2$, are values of the illumination per unit area of the portable activation panel PAN corresponding to said different values of the adjustable activation distance $d_1$ and $d_2$.

It was surprisingly found that the simultaneous optimization of the efficiency of the photocatalytic disinfection with the optimization of the performance of the irradiating lamp L is obtained within the selected ranges of the second ratio.

Should the values of the range of the second ratio $r_2$ be smaller than the above range, such as for example the range of 0.01-10 W/m2 of EP0630679, it leads to lower luminous efficacy and, possibly, too small adjustable activation distances $d$ -e.g. which may result cumbersome to use.

Should the values of the range of the second ratio $r_2$ be greater than the above range, it leads to increasing significantly the luminous efficacy and the efficiency of the photocatalytic disinfection for each activation period, which results in reducing the total number of activation hours over 24 hours, said reduction allowing microbial growth during the period when the irradiating lamp L doesn't work. Additionally, this would lead to higher electricity consumption and a shorter lifespan of the LEDs.

The minimum number of activation hours is 3 hours, said number of activation hours and said number of activation periods over 24 hours.

Said value of 3 hours has been surprisingly found to have the advantage to be applicable to all categories of hospital indoor environment. This means that the minimum number of activation hours cannot be less than 3 hours in a 24 hours period. For some specific categories of hospital indoor environment- e.g. operating room, intensive care unit, the minimum number of activation hours is greater than 3 hours, such as 4 hours, 4 hours and a half, 5 hours, or 6 hours.

According to the invention, there is at least one activation period over 24 hours. However, in most of the cases, in order to prevent microbial growth during the periods when the irradiating lamp L is powered off, it is preferable to have at least two activation periods over 24 hours, equally spaced between one another: for example the first activation period from 4 am to 8 am and the second activation period from 4pm to 8pm.

The electronic control unit ECU is arranged to carry out specific tasks based on said volume V of said hospital indoor environment, on the minimum number of activation hours, and on a selected value of the first ratio r1 and on a selected value of the second ratio $r_2$:

> to compute the area s of the portable activation panel PAN,
> to determine the adjustable activation distance $d$, the power P of the irradiating lamp L, and to send the corresponding determinations to the irradiating lamp L,
> to set each activation period, and the number of activation periods, and to send the corresponding settings to the irradiating lamp L
> to send power on instructions to the irradiating lamp L at the beginning of each activation period,
> to send power off instructions to the irradiating lamp L at the end of each activation period.

Whenever necessary, more than one medical device can be used for the same hospital indoor environment.

Panel preferred embodiments

The portable activation panel PAN can be placed anywhere within the volume V of said hospital indoor environment: on the walls, on the ceiling, on the floor, etc.

The versatile nature of the portable activation panel PAN is disclosed in several preferred embodiments.

In some panel preferred embodiments, the portable activation panel PAN is an arrangement of one or more connectable portable panels.

The connectable portable panels can be:

- standalone individual panels, as shown in Fig. 3a where for simplicity a single standalone panel PAN is represented.
- sliding panels, as shown in Fig. 3b
- folding panels, as shown in Fig. 3c
- rolled panels as shown in Fig. 3d.

In all panel preferred embodiments, the portable activation panel PAN has a panel surface that is essentially flat.

Irrespective of the panel preferred embodiments described above, the panel surface is either plain in some preferred embodiments not represented graphically, or light scattering surface, as shown in Fig. 3e, where the dimension of the elements of the light scattering surface are exaggerated in respect to the dimension of the portable activation panel PAN for better illustration of the concept.

In order to enhance the absorption of the daylight for the preferred embodiments when the portable activation panel PAN, the light scattering surface is placed such that said daylight irradiates the portable activation panel PAN.

The variety of panel preferred embodiments has the advantage of adapting the surface of portable activation panel PAN to any hospital indoor environment.

Non-limiting examples are below:

- the standalone individual panels can be pictures hanged on the walls,

- the sliding panels can be used whenever the hospital indoor environment can be extended from one room to two neighboring rooms,
- the folding panels can be folded in numerous ways or can be movable partitions used inside consultation rooms to allow the patient to undress, or separating the beds in the intensive care unit.

The rolled panels can fulfill the role of curtains for the preferred embodiments when the portable activation panel PAN is placed such that said daylight irradiate the portable activation panel PAN. During the daylight the rolled panels are rolled up, allowing the natural light to enter the hospital indoor environment and disinfect. When the daylight is gone, the rolled panels are rolled down in order to be used according to the invention.

Lamp preferred embodiments

Preferably, the irradiating lamp L is an arrangement of one or more LEDs, said one or more LEDs being configured to be individually powered on and, respectively, off upon receiving the power on instructions and, respectively, the power off instructions.
In some lamp preferred embodiments, the arrangement comprises more than one LEDs. In this case, the irradiating lamp L comprises a lamp housing used for accommodating as many LEDs as necessary.
In other lamp preferred embodiments, the arrangement comprises only one LED.
In a lamp preferred embodiment, the preferred wavelength $\lambda$ of the one or more LEDs is either within the range $355 \div 360$ nm or within the range $360 \div 365$ nm.
In an alternative lamp preferred embodiment, the preferred wavelength $\lambda$ of the one or more LEDs is within the range $365 \div 370$nm.

Lamp-panel preferred embodiments

Depending on the relative position of the arrangement of one or more LEDs in respect to the portable activation panel PAN there are lamp-panel preferred embodiments.
In one lamp-panel preferred embodiment, not represented graphically, the irradiating lamp L and the portable activation panel PAN are mechanically connectable by adjustable detachable mechanical fixing means such as a bar, a stick, etc. The purpose is to secure the activation distance $d$ in situations when it is highly possible that the activation distance $d$ is altered with the consequence of impairing the functioning of the medical device.
In another lamp-panel preferred embodiment, as shown in Fig. 2a and Fig. 2b, the arrangement of more LEDs is placed perimetrically to the portable activation panel PAN.
The perimetral arrangement is realized, in a non-limiting example, with the lamp housing accommodating each LED, whereas in other non-limiting example the lamp housing accommodates two or more LEDs. In the latter case the lamp housing can have the shape of a flexible tube, not represented graphically.
The perimetral arrangement is advantageous in situations such as but not limiting to the following:

- when the portable activation panel PAN has to be moved frequently from one hospital indoor environment to another or within the same hospital indoor environment due to rearrangement of the interior space,
- when there is a high transit in the hospital indoor environment surrounding the medical device (ex. hallways) and the presence of a large number of persons can either interfere with the radiation from the irradiating lamp L, or when there is a danger that the irradiating lamp L is torn down by the persons,
- when the portable activation panel PAN is a picture and the perimetral illumination creates an aesthetic effect.
- in case of using folding panels.

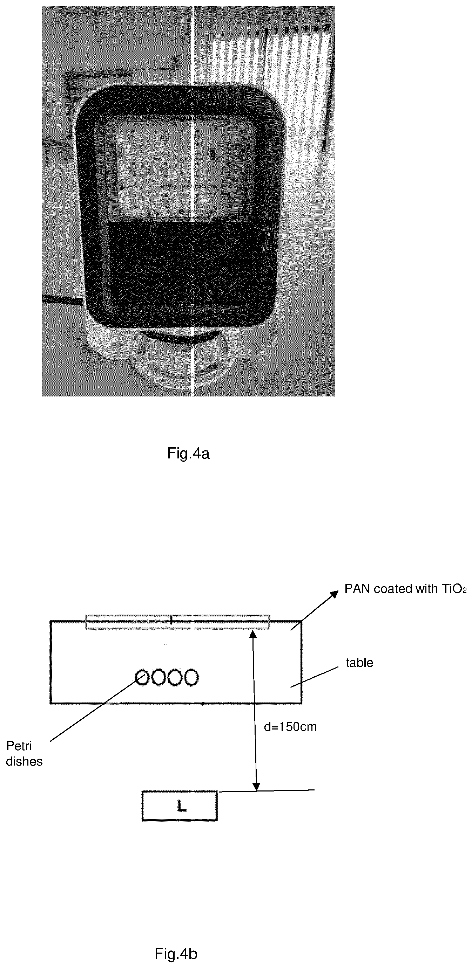

In another lamp-panel preferred embodiment, as shown in Fig. 2a and Fig.2b, the irradiating lamp L and the portable activation panel PAN are mechanically connectable by adjustable detachable mechanical fixing means. The lamp fixing means can play the role of the adjustable detachable mechanical fixing means.
In another lamp-panel preferred embodiment, the light beam falls under an adjustable activation angle $\alpha$. Said adjustable activation angle $\alpha$ is defined as the angle made by the axis of the light beam emitted by the lamp L with the surface of the portable activation panel PAN, where the value of $\alpha <$ half of the divergence angle $\theta$ of the LED.
In this case, the value of the illumination per unit area of the portable activation panel PAN of the irradiating lamp L will vary depending on the adjustable activation angle $\alpha$.
When the light beam is perpendicular on the activation panel PAN- as it is shown in Fig. 1 and Fig. 4b, the illumination per unit area of the portable activation panel PAN of the irradiating lamp L, has the highest value.
In the examples of the lamp-panel preferred embodiment, with reference to Fig. 2a and 2b, the light beam cannot be perpendicular on the activation panel PAN, because of the perimetral arrangement.

As the adjustable activation angle $\alpha$ changes, the illumination per unit area of the area s will vary depending on $cos(90°- \alpha)$.

For the same adjustable activation distance **d,** there is a direct relationship between of cos(90°- $\alpha$), and illumination per unit area of the portable activation panel PAN of the irradiating lamp L, as follows:

$$E_1/E_2 = cos\ (90°-\boldsymbol{\alpha_1})/\ cos\ (90°-\boldsymbol{\alpha_2})\ [Eq.7]$$

where $\alpha_1$ and $\alpha_2$ are two different adjustable activation angles,

and where $E_1$, respectively $E_2$ are values of illumination per unit area of the portable activation panel PAN by the irradiating lamp L, that is the measure of the luminous flux per unit area that reaches the portable activation panel PAN corresponding to said different adjustable activation angles $\alpha_1$ and $\alpha_2$.

Preferred example of realization

In the preferred example of realization, with reference to Fig. 4a and Fig. 4b, the medical device of the first alternative lamp-panel preferred embodiment is suitable for a specific volume V of a specific hospital indoor environment being in the range of $40m^3$ and $60m^3$ corresponding to specific frequently encountered categories of hospital indoor environments: patient rooms, consultation rooms, laboratories.

In the preferred example of realization:

- the portable activation panel PAN is the standalone panel having the area s around $2m^2$, corresponding to a selected value of the first ratio $r_1$ = $1m^2$ of standalone panel for each $25.2\ m^3$ of volume V. The portable activation panel PAN is a custom standalone panel made of glass having the area **s** = 2m x1m = $2m^2$ placed on a table against a wall. For simplicity reasons, it is assumed that the entire area s of the standalone panel will be irradiated. The surface of the custom standalone panel was sprayed three successive times with a sanitizer comprising titanium dioxide $TiO_2$ nanoparticles until the thin film was deposited on said custom standalone panel.
- a custom irradiating lamp L was created as a custom arrangement of 12 LEDs.

Each LED has a maximum power P = 3W, corresponding to a selected value of the second ratio $r_2$ = maximum 18 $W/m^2$,

- the lamp-panel preferred embodiment was selected, as shown in Fig. 4b,
- the preferred wavelength $\lambda$ of the LEDs is within the range 365÷370nm,
- the adjustable activation distance **d** = 1.50 m, as shown in Fig. 4b,

The details of the custom irradiating lamp L are shown below:

- type: power LEDs;
- range: UV-A;
- spectral range $\lambda$ 365÷370nm;
- divergence angle $\theta$ = 60°;
- maximum current intensity: 1000mA;
- maxim power P: 3W/LED, that is maximum power P 36W for the custom irradiating lamp L;
- size of the LEDs: 3,3x3,3x2,48mm.

The prototype of the custom irradiating lamp L is shown in Fig. 4a, the lamp housing accommodating 12 LEDs and being configured to be placed on a horizontal surface such as a table placed in front of the portable activation panel PAN, as shown in Fig. 4b.

The above details of the custom irradiating lamp L including the image of Fig. 4a must be interpreted as exemplifying the irradiating lamp L of the invention and not limiting same.

The preferred wavelength $\lambda$ of the one or more LEDs were selected with the purpose of optimizing between the wavelength, the intrinsic characteristics and advantages of using LEDs and the efficiency of the photocatalytic disinfection.

Preferred embodiment comprising photoelectric sensor

In another preferred embodiment, not represented graphically, the medical device further comprises a photoelectric sensor placed on portable activation panel PAN, the photoelectric sensor communicating with the electronic control unit ECU, the photoelectric sensor arranged to detect and measure changes in quantity of natural light entering the hospital indoor environment and to send corresponding signals to the electronic control unit ECU when said quantity of natural light is comprised within a natural light range irradiating the portable activation panel PAN said natural light range being the range of the light necessary to produce photocatalytic disinfection. The electronic control unit ECU is further arranged to store the natural light range and the values of the quantity of natural light necessary to produce photocatalytic disinfection, and to send power off instructions upon receiving the corresponding signals from said photoelectric sensor that said quantity of natural light is comprised within said natural light range said natural light range being the range of the light necessary to produce photocatalytic disinfection, which in its turn depends on the hours of daylight. The number of activation periods over 24 hours is adjusted according to the number of hours when said quantity of natural light is comprised within said natural light range.

The above-captioned preferred embodiment comprising the photoelectric sensor is suitable for those hospital indoor environments provided with one or more windows allowing daylight to penetrate through said windows, and where it is possible to arrange the position of the portable activation panel PAN facing the one or more windows, such that said daylight irradiates the portable activation panel PAN.

The hours of daylight depend on the geographical position of the facility accommodating the hospital indoor environments of interest and on the season. The values of the quantity of natural light necessary to produce photocatalytic disinfection are known from prior art.

For example, for the same specific hospital indoor environment, in the absence of the above-captioned preferred embodiment comprising the photoelectric sensor, there are two activation periods over 24 hours- say the first activation period from 9am to 1 pm and the second activation period from 9pm to 1 am, whereas in the above-captioned preferred embodiment comprising the photoelectric sensor, there is only one activation period over 24 hours, namely the second activation period from 9pm to 1 am.

The above-captioned preferred embodiment comprising the photoelectric sensor has the advantage that it allows energy saving because the irradiating lamp L is powered off during the number of hours when said quantity of natural light is comprised within said natural light range, as the number of activation periods over 24 hours is adjusted accordingly.

Combinations of embodiments

The standalone individual panels are compatible with all lamp preferred embodiments. All lamp-panel preferred embodiments are compatible with the standalone individual panels.

The sliding panels are compatible with all lamp preferred embodiments. Both alternative lamp-panel preferred embodiments are compatible with the sliding panels.

The folding panels are compatible with the lamp-panel preferred embodiment and with all lamp preferred embodiments.

The rolled panels are compatible with the lamp-panel preferred embodiment and with all lamp preferred embodiments.

The photoelectric preferred embodiment is compatible with all lamp preferred embodiments, all lamp-panel preferred embodiments and the example of realization.

The advantage of the numerous combinations of panel preferred embodiments and lamp preferred embodiments is that it offers versatility of the medical device to adapt to the destination of each specific category of hospital indoor environment and to each specific volume of the hospital indoor environment.

Experiments

Two experiments were conducted:

- the first in chronological order was carried out at the Pius Brinzeu hospital of Timisoara, hereafter called "the hospital experiment".
- the second in chronological order was carried out at The Polytechnical University of Timisoara, hereafter called "the university experiment".

Both experiments used the same sanitizer comprising non-doped titanium dioxide TiO2 nanoparticles, sprayed three successive times until the thin film was deposited on the respective panels.

The hospital experiment used a common glass panel whereas the university experiment used the custom standalone panel of the preferred example of realization.

Both experiments were conducted in respective rooms having similar volume, that is around 50 m$^3$, hereafter called the experiment room.

The hospital experiment used a wide-spectrum lamp, whereas the university experiment used the custom irradiating lamp L of the preferred example of realization.

At the end, the results of the hospital experiment were compared with the results of the university experiment.

The hospital experiment was carried out on two types of germs: i) reference bacterial strains and reference fungi belonging to the American Type Culture Collection (ATCC) recommended by CLSI (Clinical and Laboratory Standards Institute) used for testing for quality control, and ii) bacterial strains and, respectively fungi, originating from various hospital indoor environments of Pius Brinzeu hospital. The level of resistance to antibiotics for the germs included in the hospital experiment varied from germs having the natural sensitivity preserved, until to multi-resistant germs, alternatively called multidrug resistant germs MDR, characterized by resistance to more than 3 classes of antibiotics.

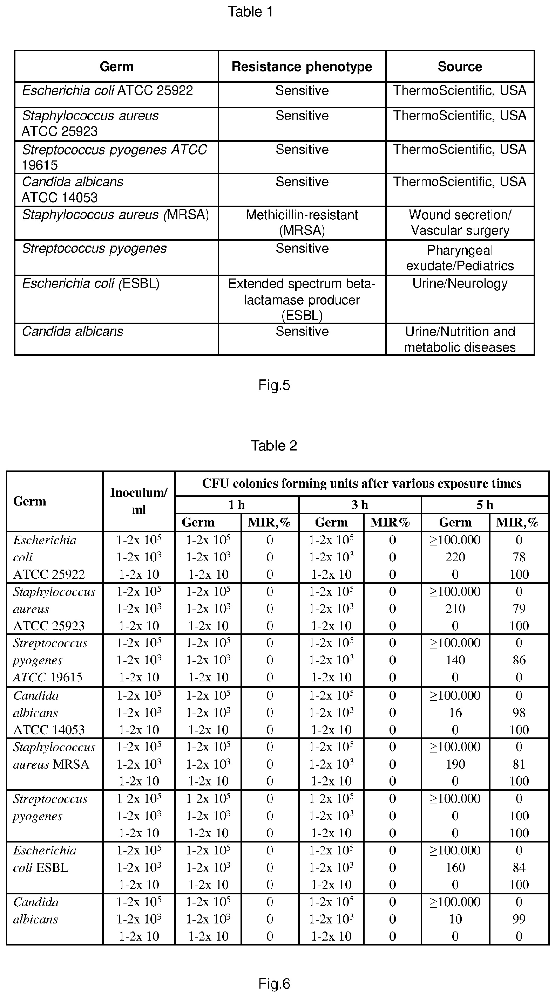

The characteristics of the germs included in the hospital experiment are presented in Fig 5 - Table 1.

For each of the germs of Table 1, suspensions in saline solution of 0.5 Mc Farland - the approximate equivalent of $1\text{-}2\text{x}10^8$ microorganisms/ml were prepared from 24-hour germs grown on three different plates: i) 24-hour bacterial strains grown on Columbia agar + 5% ram blood (COS, Certificate Croatia), ii) 24-hour bacterial strains grown on chromogenic agar (UTI, Certificate Croatia) and iii) on respectively 24-hour grown fungi on Sabouraud + chloramphenicol medium (SAB+C, Certificate Croatia). From each standardized suspension, a density of $1\text{-}2\text{x}10^8$ microorganisms/ml was used. From all the afore-mentioned suspensions, a quantity of 1 $\mu$l ($10^{-3}$ml) was dropped with the help of a microbiological loop and spread on the surface of the three afore-mentioned plates. Firstly, the afore-mentioned plates were covered with respective plate covers.

Said common glass panel, previously sprayed with said sanitizer was irradiated by a wide spectrum lamp while the afore-mentioned plates were covered with respective plate covers.

Then, the plate covers were removed. The sown uncovered plates were then irradiated by the same wide spectrum lamp, the strains being thus exposed to the photocatalytic action of $TiO_2$, for 1 hour, 3 hours and 5 hours, then they were incubated at 35-37°C, for 18-24 hours.

For each of the germs of Table 1, a control sample corresponding plate was kept in the same environmental conditions as the three afore-mentioned plates. The control samples were not exposed to the photocatalytic action of said sanitizer.

All tests were performed in duplicate.

The viability of the germs of the respective plates was monitored by reading the number of colonies forming units (CFU) using known counting methods after 1 hour, 3 hours and 5 hours of exposure to the wide spectrum lamp. Then, microbial inhibition rate MIR (%) was calculated for each germ, where microbial inhibition rate MIR (%) is defined as follows:

$$\text{MIR} = 100 - \text{microbial growth rate MGR [Eq.8]}$$

$$[\text{MIR}] = \%; [\text{MGR}] = \%$$

With reference to Fig. 6 - Table 2, the conclusions of the hospital experiment are as follows:

- The disinfection degree was proportional to the density of the germs and directly proportional to the time of exposure for each of the germs of Table 1,
- A noticeable microbial inhibition rate MIR (%) occurs only after 5 hours of exposure, as shown in Table 2- last column. The microbial inhibition rate MIR (%) after 1 and 3 hours was negligible.

In the university experiment, the reflectance/absorbance spectrum for said thin film of sanitizer comprising titanium dioxide $TiO_2$ nanoparticles applied on the custom standalone panel was determined with a Lambda 950 spectrophotometer, manufactured by PerkinElmer, using UV Win Lab 6.4.0.971 / 1.61.00 Lambda 900 software and a 150 mm integrating sphere.

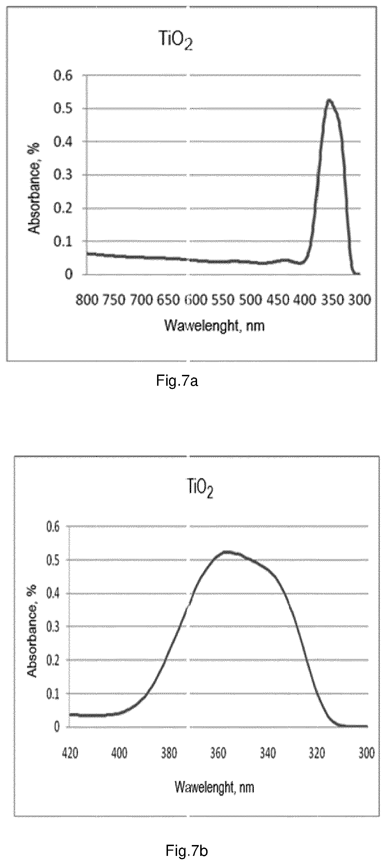

Fig. 7a and Fig. 7b illustrate the absorbance spectrum of the sanitizer comprising titanium dioxide $TiO_2$ nanoparticles according to the initial tests of the university experiment when using the same wide-spectrum lamp of the hospital experiment. Said absorbance spectrum confirms the data from earlier researches that the absorbance spectrum has the wavelength $\lambda$ in the range of 310-400 nm, with an optimum wavelength range between 320-380 nm, such as, but not limited to ranges of 355÷360 nm, or 360÷365 nm, or 365÷370nm.

The value of the forbidden energy gap is between 3 - 4 eV.

The correlation between the wavelength $\lambda$ and the energy value is:

$$\lambda = 1{,}240 / \text{E [Eq.9]}$$

$[\lambda] = \text{nm}; [E] = \text{eV}$

For each of the germs of Table 1, suspensions in saline solution of 0.5 Mc Farland were prepared from 24-hour bacterial strains grown on Columbia agar + 5% ram blood (COS, Certificate Croatia) - the approximate equivalent of 1-2x10$^8$ microorganisms/ml and, respective suspensions in saline solution of 0.5 Mc Farland were prepared from 24-hour for the grown fungi on Sabouraud + chloramphenicol medium (SAB+C, Certificate Croatia)- the approximate equivalent of 1-2x10$^8$ fungi/ml.

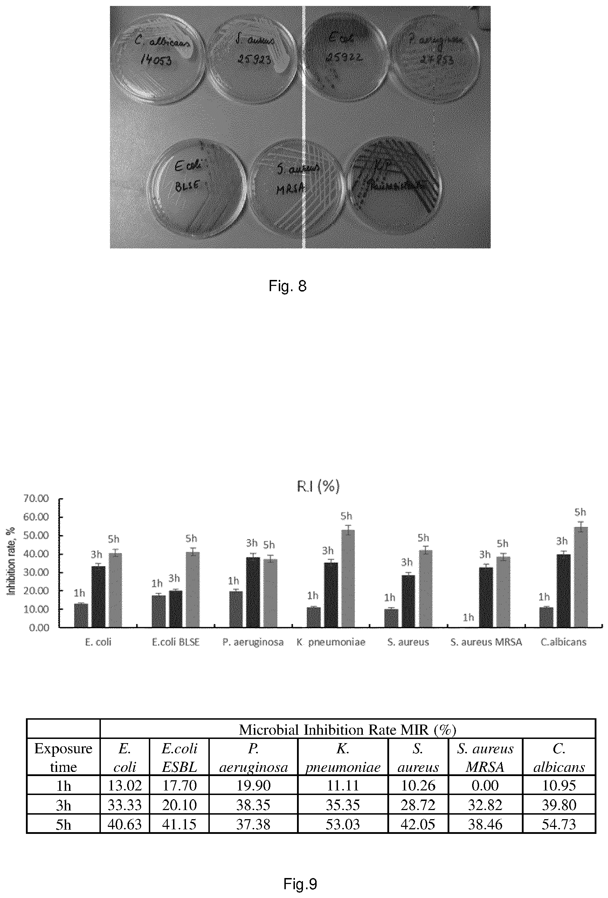

From each respective suspension corresponding to each germ, a quantity of 1 μl (10$^{-3}$ml) was dropped with the help of a microbiological loop and spread on the surface of three Petri dishes, in this way sowing approximately 1-2x10$^5$ bacteria, respectively 1-2x 10$^5$ fungi. The Petri dishes are shown in Fig. 8b.

A control sample Petri dish was kept for each of the germs of Table 1 in another space than the experiment room, outside the reach of the medical device of the preferred example of realization.

All tests were performed in duplicate.

When comparing the germs of Table 1 of the hospital experiment with the same germs of the university experiment, the difference is that in the university experiment, the germs were sown, thus encouraged to grow, whereas in the hospital environment they were left to grow without stimulation. Consequently the concentration of the germs is higher in the university experiment as compared with the hospital experiment, reason for which the university experiment addresses the most unfavorable circumstances for the health of the patients. In real-life situations, many germs find themselves in adverse conditions which do not favor their growth, whereas in the university experiment the germs were "feed" with appropriate germ food, thus had the best conditions to grow.

The medical device of the preferred example of realization was used in the experiment room where the afore-mentioned Petri dishes for each of the germs of Table 1 were placed with their respective covers removed.

The viability of the germs of the respective plates was monitored by reading the number of colonies forming units (CFU) using known counting methods after 1 hour, 3 hours and 5 hours of exposure to the custom irradiating lamp L.

The results of the university experiment are shown in Fig. 9. The conclusions of the university experiment are as follows:

- the microbial inhibition rate MIR for the respective concentration of germs varies between 0 and 18% after 1 hour, between 20 and 33% after 3 hours and between 37.38 and 54,73 after 5 hours
- after 1 hour of exposure to the custom irradiating lamp L, the smallest microbial inhibition rate MIR is the one corresponding to *S. aureus (MRSA)* namely -0% and the greatest is the one corresponding to *P.aeruginosa*- namely 19.90%.
- after 3 hours of exposure to the custom irradiating lamp L, - that is the minimum number of **activation hours,** the smallest microbial inhibition rate MIR is the one corresponding to *E. coli ESBL* 20%, and the greatest is the one corresponding to *C. albicans*-39.80%. The university experiment confirmed the minimum number of **activation hours** of using the medical device of the invention in the most unfavorable situations for the health of the patients.
- after 5 hours of exposure to the custom irradiating lamp L, the smallest microbial inhibition rate MIR is the one corresponding to *P.aeruginosa,* namely 37.38% and the greatest is the one corresponding to *C. albicans*-namely 54.73%
- the microbial inhibition rate MIR grows with the exposure time, the greatest microbial inhibition rate IR being the one corresponding to *C. albicans*-namely 54.73% after 5 hours.

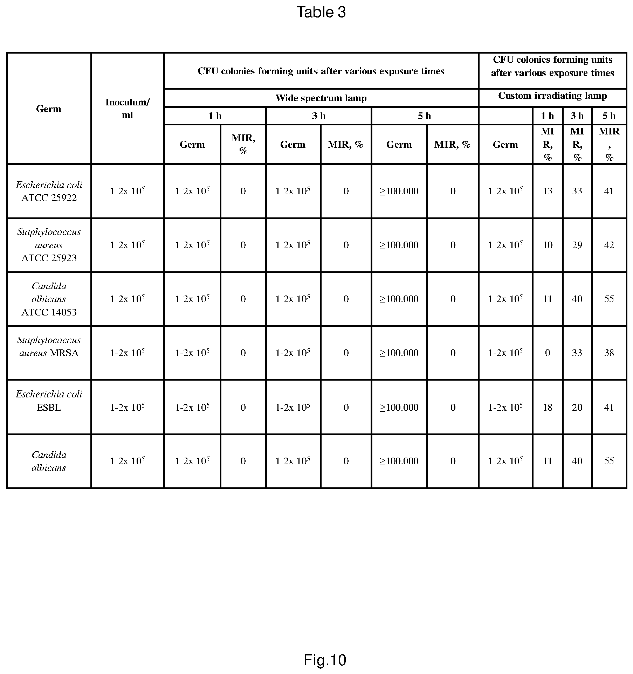

Fig. 10 Table 3 shows a comparison between the results of the hospital experiment and the university experiment for those germs that were common in both experiments.

It stems very clearly from Table 3 that, by using the medical device of the invention in its preferred example of realization, better disinfection of the hospital indoor environments is achieved as compared with prior art.

The increased level of disinfection depends on the type of germs, the best results when using the medical device of the preferred example of realization being on the *C. albicans* and *E.coli ESBL* germs.

In a second aspect of the invention it is presented a method comprising the following steps carried out by the electronic control unit ECU.

In the first step, data regarding the volume V of the hospital indoor environment contaminated with germs, the minimum number of activation hours, the selected value of the first ratio r1 and the selected value of the second ratio $r_2$ is received,

In the second step, it is computed the area s of the portable activation panel PAN by using the first ratio **$r_1$.**

In the third step, the adjustable activation distance **d,** the power P of the irradiating lamp L are determined, and the corresponding determinations are sent to the irradiating lamp L,

In the fourth step, each activation period and the number of the activation periods are set, and the corresponding settings are sent to the irradiating lamp L,

In the fifth step, the irradiating lamp L is powered on at the beginning of each activation period,

In the sixth step, the irradiating lamp L is powered off at the end of each activation period.

Taking the preferred example of realization, the steps of the method are as follows:
Before starting the method, the wavelength $\lambda$ of the one or more LEDs is selected.

In the first step, knowing the volume V of the specific hospital indoor environment, e.g. around $50m^3$ and the destination of the specific hospital indoor environment - e.g. patient room, the following parameters are inputted to the electronic control unit ECU:

- the selected first ratio $r_1 = 1m^2$ of standalone panel for each $25.2\ m^3$ of volume V,
- the minimum number of activation hours = 3 hours,
- the selected second ratio $r_2 = 18\ W/m^2$,

In the second step, the area $s$ of the portable activation panel PAN is computed by using the first ratio $r_1$: $s =$ approximately $2m^2$.

In the third step, the adjustable activation distance $d$ and the power P are determined, and the corresponding determinations are sent to the custom irradiating lamp L:

- the adjustable activation distance $d$ = 1.50 m,
- the total power P of the irradiating lamp L = $r_2$ x $s$ = 36 W, corresponding to 12 LEDs, each of them having 3W.

In the fourth step, each activation period is set to four hours and the number of the activation periods is set to two, and the corresponding settings are sent to the custom irradiating lamp L.

In the fifth step, the custom irradiating lamp L is powered on, and in the sixth step, the custom irradiating lamp L is powered off.

For the embodiments making use of the photoelectric sensor, further sub-steps are comprised after the sixth step:
In step 7-1, a first signal is received from the photoelectric sensor that said quantity of natural light is comprised within said natural light range.

In step 7-2, power off instructions are sent to the irradiating lamp L.

In step 7-3, a second signal is received from the photoelectric sensor that said quantity of natural light is not comprised within said natural light range.

In step 7-4, power on instructions are sent to the irradiating lamp L.

In step 7-5, the number of activation periods over 24 hours is adjusted according to the number of hours when said quantity of natural light is comprised within said natural light range.

In a <u>third</u> aspect of the invention, it is presented a non-transitory computer-readable medium comprising instructions which, when the program is executed by the electronic control unit ECU of the invention, causes the electronic control unit ECU to carry out the steps of the method according to any preferred embodiment.

While certain embodiments of the present invention have been described in detail, those skilled in the art will recognize various alternative embodiments for practicing the invention as defined by the following claims.

**Claims**

1. A medical device suitable for disinfecting a volume V of a hospital indoor environment contaminated with germs, the medical device comprising a portable activation panel PAN, having a non-porous portable activation panel PAN surface coated with a film of titanium dioxide TiO2 nanoparticles, arranged to be maintained firmly in place during said disinfecting, the portable activation panel PAN acting as photocatalyst, one irradiating lamp L, and an electronic control unit ECU,

    the irradiating lamp L being placed at an adjustable activation distance $d$ from the portable activation panel PAN, the irradiating lamp L arranged to be maintained firmly in place during said disinfecting,
    the irradiating lamp L comprising at least one LED (Light Emitting Diode) having a wavelength $\lambda$ in the range of 320-380 nm and a power P, the irradiating lamp L being arranged to irradiate the portable activation panel PAN during at least one activation period over 24 hours, where each activation period is equal to or greater than a minimum number of activation hours of 3 hours,
    the electronic control unit ECU being in communication with the irradiating lamp L, the irradiating lamp L being operable by the electronic control unit ECU,
    wherein
    a first ratio $r_1$ is defined between an area $s$ of the portable activation panel PAN and the volume V of said hospital indoor environment, said first ratio $r_1$ being in the range of 1:20 - 1:30,

a second ratio $r_2$ is defined as the power of said irradiating lamp transferred per unit area of the portable activation panel PAN, said second ratio $r_2$ being in the range of 16.5 - 19.5 W/m$^2$,

wherein

based on said volume V of said hospital indoor environment, on the minimum number of activation hours, and on a selection of the first ratio $r_1$ and of the second ratio $r_2$, the electronic control unit ECU is arranged:

> to compute the area s of the portable activation panel PAN,
> to determine the adjustable activation distance **d**, the power P of the irradiating lamp L, and to send the corresponding determinations to the irradiating lamp L,
> to set each activation period and the number of activation periods, and to send the corresponding settings to the irradiating lamp L,
> to send power on instructions to the irradiating lamp L at the beginning of each activation period,
> to send power off instructions to the irradiating lamp L at the end of each activation period.

wherein

> the medical device is configured to carry out photocatalytic disinfection of said V of said hospital indoor environment for each activation period by irradiating the area s of the portable activation panel PAN by the irradiating lamp L,
> **characterized in that** said portable activation panel PAN is a sliding panel, a folding panel, or a panel configured to be rolled up and down.

2. The medical device of any of the claim 1, wherein the portable activation panel PAN is an arrangement of one or more connectable portable panels.

3. The medical device of any preceding claim, wherein the irradiating lamp L is preferably an arrangement of one or more LEDs, said one or more LEDs being configured to be individually powered on and, respectively, off upon receiving the power on instructions and, respectively, the power off instructions.

4. The medical device of claim 3, wherein the preferred wavelength $\lambda$ of the one or more LEDs is either within the range 355-360 nm or within the range 360-365 nm.

5. The medical device of any of claim 3, wherein the preferred wavelength $\lambda$ of the one or more LEDs is within the range 365-370 nm.

6. The medical device of any of the claims 3 to 5 , wherein the arrangement of more LEDs is placed perimetrically to the portable activation panel PAN.

7. The medical device of claim 5 suitable for a specific volume V of a specific hospital indoor environment in the range of 40m$^3$ and 60m$^3$, wherein

> the portable activation panel PAN is the standalone panel having the area **s** about 2m$^2$,
> the arrangement of LEDs comprises 12 LEDs, each LED having the power P = 3W, the preferred wavelength $\lambda$ of the LEDs is within the range 365-370 nm,
> the adjustable activation distance **d** = 1.50 m,

8. The medical device of any preceding claim, wherein

> the medical device further comprises a photoelectric sensor placed on portable activation panel PAN, the photoelectric sensor communicating with the electronic control unit ECU, the photoelectric sensor arranged to detect and measure changes in quantity of natural light entering the hospital indoor environment and to send corresponding signals to the electronic control unit ECU when said quantity of natural light is comprised within a natural light range irradiating the portable activation panel PAN, said natural light range being the range of the light necessary to produce photocatalytic disinfection,
> the electronic control unit ECU is further arranged to store the natural light range and the values of the quantity of natural light necessary to produce photocatalytic disinfection, and to send power off instructions upon receiving the corresponding signals from said photoelectric sensor that said quantity of natural light is comprised within said natural light range.

the number of activation periods over 24 hours is adjusted according to the number of hours when said quantity of natural light is comprised within said natural light range.

9. A computer-implemented method of configuration and operation of the medical device of any of the claims 1 to 8, comprising the following steps carried out by the electronic control unit ECU:

S1 receiving data regarding the volume V of the hospital indoor environment contaminated with germs, the minimum number of activation hours, the selected value of the first ratio $r_1$ and the selected value of the second ratio $r_2$:

S2 computing the area s of the portable activation panel PAN by using the first ratio,

S3 determining the adjustable activation distance $d$, the power P of the irradiating lamp L, and sending the corresponding determinations to the irradiating lamp L,

S4 setting each activation period and the number of the activation periods, and sending the corresponding settings to the irradiating lamp L,

S5 powering on the irradiating lamp L at the beginning of each activation period,

S6 powering off the irradiating lamp L at the end of each activation period.

10. The computer-implemented method of claim 9 , wherein further sub-steps are comprised after step S6:

S7-1 receiving a first signal from the photoelectric sensor that said quantity of natural light is comprised within said natural light range,

S7-2 sending power off instructions to the irradiating lamp L,

S7-3 receiving a second signal from the photoelectric sensor that said quantity of natural light is not comprised within said natural light range,

S7-4 sending power on instructions to the irradiating lamp L,

S7-5 adjusting the number of activation periods over 24 hours according to the number of hours when said light quantity of natural light is comprised within said natural light range.

11. A non-transitory computer-readable medium comprising instructions which, when the program is executed by the electronic control unit ECU of claim 9 or 10, causes the electronic control unit ECU to carry out the steps of the method according to any of the claims 1 to 8.

## Patentansprüche

1. Medizinische Vorrichtung, die zum Desinfizieren eines mit Keimen kontaminierten Volumens V einer Krankenhausinnenumgebung geeignet ist, wobei die medizinische Vorrichtung ein tragbares Aktivierungspanel PAN, das eine nicht poröse tragbare Aktivierungspanel- PAN Oberfläche aufweist, die mit einem Film aus Titandioxid-, $TiO_2$, Nanopartikeln beschichtet ist und dazu angeordnet ist, während des Desinfizierens fest an ihrem Platz gehalten zu werden, wobei das tragbare Aktivierungspanel PAN als Photokatalysator fungiert, eine Bestrahlungslampe L und eine elektronische Steuereinheit ECU umfasst,

wobei die Bestrahlungslampe L in einem einstellbaren Aktivierungsabstand $d$ von dem tragbaren Aktivierungspanel PAN platziert ist, wobei die Bestrahlungslampe L dazu angeordnet ist, während des Desinfizierens fest an ihrem Platz gehalten zu werden,

wobei die Bestrahlungslampe L mindestens eine LED (Leuchtdiode) umfasst, die eine Wellenlänge λ im Bereich von 320-380 nm und eine Leistung P aufweist,

wobei die Bestrahlungslampe L dazu angeordnet ist, das tragbare Aktivierungspanel PAN während mindestens einer Aktivierungsperiode über 24 Stunden zu bestrahlen, wobei jede Aktivierungsperiode gleich oder größer als eine Mindestanzahl von Aktivierungsstunden von 3 Stunden ist,

wobei die elektronische Steuereinheit ECU mit der Strahlungslampe L in Kommunikation steht,

wobei die Strahlungslampe L durch die elektronische Steuereinheit ECU bedienbar ist,

wobei

ein erstes Verhältnis $r_1$ zwischen einer Fläche **s** des tragbaren Aktivierungspaneels PAN und dem Volumen V der Krankenhausinnenumgebung definiert ist, wobei das erste Verhältnis $r_1$ im Bereich von 1:20 - 1:30 liegt,

ein zweites Verhältnis $r_2$ als die Leistung der Strahlungslampe definiert ist, die pro Einheitsfläche des tragbaren Aktivierungspanels PAN übertragen wird, wobei das zweite Verhältnis $r_2$ im Bereich von 16,5 - 19,5 $W/m^2$ liegt,

wobei

basierend auf dem Volumen V der Krankenhausinnenumgebung, der Mindestanzahl von Aktivierungsstunden und einer Auswahl des ersten Verhältnisses $r_1$ und des zweiten Verhältnisses $r_2$ die elektronische Steuereinheit ECU für Folgendes angeordnet wird:

die Flächen s des tragbaren Aktivierungspanels PAN zu berechnen,
den einstellbaren Aktivierungsabstand $d$ und die Leistung P der Bestrahlungslampe L zu bestimmen und die entsprechenden Bestimmungen an die Bestrahlungslampe L zu senden,
jede Aktivierungsperiode und die Anzahl der Aktivierungsperioden einzustellen und die entsprechenden Einstellungen an die Bestrahlungslampe L zu senden,
zum Beginn jeder Aktivierungsperiode Einschaltanweisungen an die Strahlungslampe L zu senden,
um am Ende jeder Aktivierungsperiode Ausschaltanweisungen an die Strahlungslampe L zu senden;
wobei
die medizinische Vorrichtung dazu konfiguriert ist, für jede Aktivierungsperiode eine photokatalytische Desinfektion des V der Krankenhausinnenumgebung auszuführen, indem sie den Bereich s des tragbaren Aktivierungspanels PAN mit der Bestrahlungslampe L bestrahlt,
**dadurch gekennzeichnet, dass** das tragbare Aktivierungspanel PAN ein Schiebepanel, ein Faltpaneel oder ein zum Auf- und Abrollen konfiguriertes Panel ist.

2. Medizinische Vorrichtung nach Anspruch 1, wobei das tragbare Aktivierungspanel PAN eine Anordnung eines oder mehrerer verbindbarer tragbarer Panels ist.

3. Medizinische Vorrichtung nach einem vorstehenden Anspruch, wobei die Bestrahlungslampe L vorzugsweise eine Anordnung einer oder mehrerer LEDs ist, wobei die eine oder mehreren LEDs dazu konfiguriert sind, beim Empfangen der Einschaltanweisungen bzw. der Ausschaltanweisungen einzeln ein- bzw. ausgeschaltet zu werden.

4. Medizinische Vorrichtung nach Anspruch 3, wobei die bevorzugte Wellenlänge $\lambda$ der einen oder mehreren LEDs entweder im Bereich von 355-360 nm oder im Bereich von 360-365 nm liegt.

5. Medizinische Vorrichtung nach Anspruch 3, wobei die bevorzugte Wellenlänge $\lambda$ der einen oder mehreren LEDs im Bereich von 365-370 nm liegt.

6. Medizinische Vorrichtung nach einem der Ansprüche 3 bis 5, wobei die Anordnung mehrerer LEDs perimetrisch zum tragbaren Aktivierungspanel PAN platziert ist.

7. Medizinische Vorrichtung nach Anspruch 5, die für ein spezifisches Volumen V einer spezifischen Krankenhausinnenumgebung im Bereich von 40m$^3$ bis 60m$^3$ geeignet ist, wobei

das tragbare Aktivierungspanel PAN das eigenständige Panel ist, das die Fläche s von etwa 2m$^2$ aufweist,
die Anordnung von LEDs 12 LEDs umfasst, wobei jede LED die Leistung P = 3W aufweist,
die bevorzugte Wellenlänge $\lambda$ der LEDs im Bereich von 365-370 nm liegt,
der einstellbare Aktivierungsabstand $d =$ 1,50 m,

8. Vorrichtung nach einem vorstehenden Anspruch, wobei

die medizinische Vorrichtung weiter einen photoelektrischen Sensor umfasst, der auf dem tragbaren Aktivierungspanel PAN platziert ist, wobei der photoelektrische Sensor mit der elektronischen Steuereinheit ECU kommuniziert, der photoelektrische Sensor dazu angeordnet ist, Änderungen der Menge an natürlichem Licht, das in die Krankenhausinnenumgebung gelangt, erkennt und misst und entsprechende Signale an die elektronische Steuereinheit ECU zu senden, wenn die Menge an natürlichem Licht innerhalb eines natürlichen Lichtbereichs umfasst ist, der das tragbare Aktivierungspanel PAN bestrahlt, wobei der natürliche Lichtbereich der Bereich des Lichts ist, der zur Erzeugung einer photokatalytischen Desinfektion erforderlich ist,
die elektronische Steuereinheit ECU weiter dazu angeordnet ist, den natürlichen Lichtbereich und die Werte der Menge an natürlichem Licht zu speichern, die zur Erzeugung einer photokatalytischen Desinfektion erforderlich ist, und Ausschaltanweisungen zu senden, wenn sie die entsprechenden Signale von dem photoelektrischen Sensor empfängt, dass die Menge an natürlichem Licht innerhalb des natürlichen Lichtbereichs umfasst ist,
die Anzahl von Aktivierungsperioden über 24 Stunden gemäß der Anzahl von Stunden angepasst wird, in denen die Menge an natürlichem Licht innerhalb des natürlichen Lichtbereichs umfasst ist.

9. Computerimplementiertes Verfahren zur Konfiguration und zum Betrieb der medizinischen Vorrichtung nach einem der Ansprüche 1 bis 8, das die folgenden Schritte umfasst, die von der elektronischen Steuereinheit ECU ausgeführt werden:

S1 Empfangen von Daten über das Volumen V der mit Keimen kontaminierten Krankenhausinnenumgebung, die Mindestanzahl von Aktivierungsstunden, den ausgewählten Wert des ersten Verhältnisses $r_1$ und den ausgewählten Wert des zweiten Verhältnisses $r_2$:

S2 Berechnen der Fläche $s$ des tragbaren Aktivierungspanels PAN unter Verwendung des ersten Verhältnisses,
S3 Bestimmen des einstellbaren Aktivierungsabstands $d$, der Leistung P der Bestrahlungslampe L und Senden der entsprechenden Bestimmungen an die Bestrahlungslampe L,
S4 Einstellen jeder Aktivierungsperiode und der Anzahl der Aktivierungsperioden und Senden der entsprechenden Einstellungen an die Bestrahlungslampe L,
S5 Einschalten der Bestrahlungslampe L zu Beginn jeder Aktivierungsperiode,
S6 Ausschalten der Bestrahlungslampe L am Ende jeder Aktivierungsperiode.

10. Computerimplementiertes Verfahren nach Anspruch 9, wobei nach Schritt S6 weitere Teilschritte umfasst sind:

S7-1 Empfangen eines ersten Signals vom photoelektrischen Sensor, dass die Menge an natürlichem Licht innerhalb des natürlichen Lichtbereichs umfasst ist,
S7-2 Senden von Ausschaltanweisungen an die Bestrahlungslampe L,
S7-3 Empfangen eines zweiten Signals vom photoelektrischen Sensor, dass die Menge an natürlichem Licht nicht innerhalb des natürlichen Lichtbereichs umfasst ist,
S7-4 Senden von Einschaltanweisungen an die Bestrahlungslampe L,
S7-5 Anpassen der Anzahl von Aktivierungsperioden über 24 Stunden gemäß der Anzahl von Stunden, in denen die Menge an natürlichem Licht innerhalb des natürlichen Lichtbereichs umfasst ist.

11. Nichtflüchtiges, computerlesbares Medium, das Anweisungen umfasst, die, wenn das Programm von der elektronischen Steuereinheit ECU nach Anspruch 9 oder 10 ausgeführt wird, die elektronische Steuereinheit ECU dazu veranlassen, die Schritte des Verfahrens nach einem der Ansprüche 1 bis 8 auszuführen.

## Revendications

1. Dispositif médical adapté à la désinfection d'un volume V d'un environnement intérieur hospitalier contaminé par des germes, le dispositif médical comprenant un panneau d'activation portable PAN, présentant une surface PAN de panneau d'activation portable non poreuse revêtue d'un film de nanoparticules de dioxyde de titane $TiO_2$, agencé pour être maintenu fermement en place pendant ladite désinfection, le panneau d'activation portable PAN agissant en tant que photocatalyseur, une lampe irradiante L et une unité de commande électronique ECU,

la lampe irradiante L étant placée à une distance d'activation réglable $d$ du panneau d'activation portable PAN, la lampe irradiante L étant agencée pour être maintenue fermement en place pendant ladite désinfection,
la lampe irradiante L comprenant au moins une LED (diode électroluminescente) présentant une longueur d'onde λ dans la plage de 320-380 nm et une puissance P,
la lampe irradiante L étant agencée pour irradier le panneau d'activation portable PAN pendant au moins une période d'activation sur 24 heures, où chaque période d'activation est égale ou supérieure à un nombre minimum d'heures d'activation de 3 heures,
l'unité de commande électronique ECU étant en communication avec la lampe irradiante L,
la lampe irradiante L étant actionnable par l'unité de commande électronique ECU,
dans lequel
un premier rapport $r_1$ est défini entre une surface $s$ du panneau d'activation portable PAN et le volume V dudit environnement intérieur hospitalier, ledit premier rapport $r_1$ étant dans la plage de 1:20 - 1:30,
un second rapport $r_2$ est défini comme la puissance de ladite lampe irradiante transférée par unité de surface du panneau d'activation portable PAN, ledit second rapport $r_2$ étant dans la plage de 16,5 - 19,5 $W/m^2$,
dans lequel
sur la base du volume V dudit environnement intérieur hospitalier, du nombre minimum d'heures d'activation et d'une sélection du premier rapport $r_1$ et du second rapport $r_2$, l'unité de commande électronique ECU est agencée :

pour calculer les surfaces du panneau d'activation portable PAN,

pour déterminer la distance d'activation réglable $d$, la puissance P de la lampe irradiante L, et pour envoyer les déterminations correspondantes à la lampe irradiante L,

pour régler chaque période d'activation et le nombre de périodes d'activation, et pour envoyer les réglages correspondants à la lampe irradiante L,

pour envoyer des instructions de mise sous tension à la lampe irradiante L au début de chaque période d'activation,

pour envoyer des instructions de mise hors tension à la lampe irradiante L à la fin de chaque période d'activation,

dans lequel

le dispositif médical est configuré pour réaliser une désinfection photocatalytique dudit V dudit environnement intérieur hospitalier pour chaque période d'activation en irradiant la zone s du panneau d'activation portable PAN par la lampe irradiante L,

**caractérisé en ce que** ledit panneau d'activation portable PAN est un panneau coulissant, un panneau pliant ou un panneau configuré pour être enroulé en haut et en bas.

2. Dispositif médical selon l'une quelconque des revendications 1, dans lequel le panneau d'activation portable PAN est un agencement d'un ou de plusieurs panneaux portables connectables.

3. Dispositif médical selon une quelconque revendication précédente, dans lequel la lampe irradiante L est de préférence un agencement d'une ou de plusieurs LED, lesdites une ou plusieurs LED étant configurées pour être individuellement mises sous tension et, respectivement, mises hors tension lors de la réception des instructions de mise sous tension et, respectivement, des instructions de mise hors tension.

4. Dispositif médical selon la revendication 3, dans lequel la longueur d'onde préférée $\lambda$ de l'une ou des plusieurs LED est soit au sein de la plage 355-360 nm, soit au sein de la plage 360-365 nm.

5. Dispositif médical selon l'une quelconque des revendications 3, dans lequel la longueur d'onde préférée $\lambda$ de l'une ou des plusieurs LED est au sein de la plage 365-370 nm.

6. Dispositif médical selon l'une quelconque des revendications 3 à 5, dans lequel l'agencement de plusieurs LED est placé de manière périmétrique par rapport au panneau d'activation portable PAN.

7. Dispositif médical selon la revendication 5, adapté à un volume V spécifique d'un environnement intérieur hospitalier spécifique dans la plage de 40 m³ et 60 m³, dans lequel

le panneau d'activation portable PAN est le panneau autonome présentant la surface s d'environ 2 m²,

l'agencement de LED comprend 12 LED, chaque LED présentant une puissance P = 3W,

la longueur d'onde préférée $\lambda$ des LED est au sein de la plage 365-370 nm,

la distance d'activation ajustable $d$ = 1,50 m,

8. Dispositif médical selon l'une quelconque des revendications précédentes, dans lequel

le dispositif médical comprend en outre un capteur photoélectrique placé sur le panneau d'activation portable PAN, le capteur photoélectrique communiquant avec l'unité de commande électronique ECU, le capteur photoélectrique étant agencé pour détecter et mesurer des changements de quantité de lumière naturelle entrant dans l'environnement intérieur hospitalier et pour envoyer des signaux correspondants à l'unité de commande électronique ECU lorsque ladite quantité de lumière naturelle est comprise au sein d'une plage de lumière naturelle irradiant le panneau d'activation portable PAN, ladite plage de lumière naturelle étant la plage de la lumière nécessaire pour produire une désinfection photocatalytique,

l'unité de commande électronique ECU est en outre agencée pour stocker la plage de lumière naturelle et les valeurs de la quantité de lumière naturelle nécessaire pour produire une désinfection photocatalytique, et pour envoyer des instructions de mise hors tension lors de la réception des signaux correspondants dudit capteur photoélectrique que ladite quantité de lumière naturelle est comprise au sein de ladite plage de lumière naturelle, le nombre de périodes d'activation sur 24 heures est ajusté conformément au nombre d'heures pendant lesquelles ladite quantité de lumière naturelle est comprise au sein de ladite plage de lumière naturelle.

9. Procédé mis en œuvre par ordinateur de configuration et de fonctionnement du dispositif médical selon l'une quelconque des revendications 1 à 8, comprenant les étapes suivantes mises en œuvre par l'unité de commande électronique ECU :

S1 recevoir des données concernant le volume V de l'environnement intérieur hospitalier contaminé par des germes, le nombre minimum d'heures d'activation, la valeur sélectionnée du premier rapport $r_1$ et la valeur sélectionnée du second rapport $r_2$ :

S2 calculer la surface **s** du panneau d'activation portable PAN en utilisant le premier rapport,

S3 déterminer la distance d'activation réglable **d,** la puissance P de la lampe irradiante L, et envoyer les déterminations correspondantes à la lampe irradiante L,

S4 régler chaque période d'activation et le nombre des périodes d'activation, et envoyer les réglages correspondants à la lampe irradiante L,

S5 mettre sous tension la lampe irradiante L au début de chaque période d'activation,

S6 mettre hors tension la lampe irradiante L à la fin de chaque période d'activation.

10. Procédé mis en œuvre par ordinateur selon la revendication 9, dans lequel des sous-étapes supplémentaires sont comprises après l'étape S6 :

S7-1 recevoir un premier signal du capteur photoélectrique que ladite quantité de lumière naturelle est comprise au sein de ladite plage de lumière naturelle,

S7-2 envoyer des instructions de mise hors tension à la lampe irradiante L,

S7-3 recevoir un second signal du capteur photoélectrique que ladite quantité de lumière naturelle n'est pas comprise au sein de ladite plage de lumière naturelle,

S7-4 envoyer des instructions de mise sous tension à la lampe irradiante L,

S7-5 ajuster le nombre de périodes d'activation sur 24 heures conformément au nombre d'heures pendant lesquelles ladite quantité de lumière naturelle est comprise au sein de ladite plage de lumière naturelle.

11. Support lisible par ordinateur non transitoire comprenant des instructions qui, lorsque le programme est exécuté par l'unité de commande électronique ECU selon la revendication 9 ou 10, amène l'unité de commande électronique ECU à mettre en œuvre les étapes du procédé selon l'une quelconque des revendications 1 à 8.

PAN coated with TiO$_2$

Fig.1

Fig.2a

Fig.2b

Fig.3a

Fig.3C

sliding
direction

Fig.3b

Fig.3d

PAN coated
with TiO2

light scattering
surface
of panel

Fig.3e

Fig.4a

PAN coated with TiO$_2$

table

d=150cm

Petri
dishes

L

Fig.4b

Table 1

| Germ | Resistance phenotype | Source |
|---|---|---|
| *Escherichia coli* ATCC 25922 | Sensitive | ThermoScientific, USA |
| *Staphylococcus aureus* ATCC 25923 | Sensitive | ThermoScientific, USA |
| *Streptococcus pyogenes* ATCC 19615 | Sensitive | ThermoScientific, USA |
| *Candida albicans* ATCC 14053 | Sensitive | ThermoScientific, USA |
| *Staphylococcus aureus* (MRSA) | Methicillin-resistant (MRSA) | Wound secretion/ Vascular surgery |
| *Streptococcus pyogenes* | Sensitive | Pharyngeal exudate/Pediatrics |
| *Escherichia coli* (ESBL) | Extended spectrum beta-lactamase producer (ESBL) | Urine/Neurology |
| *Candida albicans* | Sensitive | Urine/Nutrition and metabolic diseases |

Fig.5

Table 2

| Germ | Inoculum/ ml | CFU colonies forming units after various exposure times | | | | | |
|---|---|---|---|---|---|---|---|
| | | 1 h | | 3 h | | 5 h | |
| | | Germ | MIR,% | Germ | MIR% | Germ | MIR,% |
| *Escherichia coli* ATCC 25922 | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 220 | 78 |
| | $1\text{-}2\times 10$ | $1\text{-}2\times 10$ | 0 | $1\text{-}2\times 10$ | 0 | 0 | 100 |
| *Staphylococcus aureus* ATCC 25923 | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 210 | 79 |
| | $1\text{-}2\times 10$ | $1\text{-}2\times 10$ | 0 | $1\text{-}2\times 10$ | 0 | 0 | 100 |
| *Streptococcus pyogenes* ATCC 19615 | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 140 | 86 |
| | $1\text{-}2\times 10$ | $1\text{-}2\times 10$ | 0 | $1\text{-}2\times 10$ | 0 | 0 | 0 |
| *Candida albicans* ATCC 14053 | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 16 | 98 |
| | $1\text{-}2\times 10$ | $1\text{-}2\times 10$ | 0 | $1\text{-}2\times 10$ | 0 | 0 | 100 |
| *Staphylococcus aureus* MRSA | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 190 | 81 |
| | $1\text{-}2\times 10$ | $1\text{-}2\times 10$ | 0 | $1\text{-}2\times 10$ | 0 | 0 | 100 |
| *Streptococcus pyogenes* | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 0 | 100 |
| | $1\text{-}2\times 10$ | $1\text{-}2\times 10$ | 0 | $1\text{-}2\times 10$ | 0 | 0 | 100 |
| *Escherichia coli* ESBL | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 160 | 84 |
| | $1\text{-}2\times 10$ | $1\text{-}2\times 10$ | 0 | $1\text{-}2\times 10$ | 0 | 0 | 100 |
| *Candida albicans* | $1\text{-}2\times 10^5$ | $1\text{-}2\times 10^5$ | 0 | $1\text{-}2\times 10^5$ | 0 | $\geq 100.000$ | 0 |
| | $1\text{-}2\times 10^3$ | $1\text{-}2\times 10^3$ | 0 | $1\text{-}2\times 10^3$ | 0 | 10 | 99 |
| | $1\text{-}2\times 10$ | $1\text{-}2\times 10$ | 0 | $1\text{-}2\times 10$ | 0 | 0 | 0 |

Fig.6

Fig.7a

Fig.7b

Fig. 8

Fig.9

| Exposure time | Microbial Inhibition Rate MIR (%) | | | | | | |
|---|---|---|---|---|---|---|---|
| | *E. coli* | *E.coli ESBL* | *P. aeruginosa* | *K. pneumoniae* | *S. aureus* | *S. aureus MRSA* | *C. albicans* |
| 1h | 13.02 | 17.70 | 19.90 | 11.11 | 10.26 | 0.00 | 10.95 |
| 3h | 33.33 | 20.10 | 38.35 | 35.35 | 28.72 | 32.82 | 39.80 |
| 5h | 40.63 | 41.15 | 37.38 | 53.03 | 42.05 | 38.46 | 54.73 |

Table 3

| Germ | Inoculum/ ml | CFU colonies forming units after various exposure times | | | | | | CFU colonies forming units after various exposure times | | | |
|---|---|---|---|---|---|---|---|---|---|---|---|
| | | Wide spectrum lamp | | | | | | Custom irradiating lamp | | | |
| | | 1 h | | 3 h | | 5 h | | | 1 h | 3 h | 5 h |
| | | Germ | MIR, % | Germ | MIR, % | Germ | MIR, % | Germ | MIR, % | MIR, % | MIR, % |
| *Escherichia coli* ATCC 25922 | $1\text{-}2x\ 10^5$ | $1\text{-}2x\ 10^5$ | 0 | $1\text{-}2x\ 10^5$ | 0 | $\geq100.000$ | 0 | $1\text{-}2x\ 10^5$ | 13 | 33 | 41 |
| *Staphylococcus aureus* ATCC 25923 | $1\text{-}2x\ 10^5$ | $1\text{-}2x\ 10^5$ | 0 | $1\text{-}2x\ 10^5$ | 0 | $\geq100.000$ | 0 | $1\text{-}2x\ 10^5$ | 10 | 29 | 42 |
| *Candida albicans* ATCC 14053 | $1\text{-}2x\ 10^5$ | $1\text{-}2x\ 10^5$ | 0 | $1\text{-}2x\ 10^5$ | 0 | $\geq100.000$ | 0 | $1\text{-}2x\ 10^5$ | 11 | 40 | 55 |
| *Staphylococcus aureus* MRSA | $1\text{-}2x\ 10^5$ | $1\text{-}2x\ 10^5$ | 0 | $1\text{-}2x\ 10^5$ | 0 | $\geq100.000$ | 0 | $1\text{-}2x\ 10^5$ | 0 | 33 | 38 |
| *Escherichia coli* ESBL | $1\text{-}2x\ 10^5$ | $1\text{-}2x\ 10^5$ | 0 | $1\text{-}2x\ 10^5$ | 0 | $\geq100.000$ | 0 | $1\text{-}2x\ 10^5$ | 18 | 20 | 41 |
| *Candida albicans* | $1\text{-}2x\ 10^5$ | $1\text{-}2x\ 10^5$ | 0 | $1\text{-}2x\ 10^5$ | 0 | $\geq100.000$ | 0 | $1\text{-}2x\ 10^5$ | 11 | 40 | 55 |

Fig.10

## REFERENCES CITED IN THE DESCRIPTION

**Patent documents cited in the description**

- EP 0630679 A

- US 2016249760 A